# FASCICULE DE BREVET EUROPEEN

(11) **EP 2 294 648 B1**
(45) Date de publication et mention de la délivrance du brevet: **15.03.2017**
(21) Numéro de dépôt: 09766061.7
(22) Date de dépôt: 27.05.2009
(51) Int. Cl.: H01M 8/16

(54) **PRODUCTION D'UN BIOFILM SUR UNE ELECTRODE POUR BIOPILE, ELECTRODE ET BIOPILE OBTENUES**
HERSTELLUNG EINER BIOSCHICHT AUF EINER ELEKTRODE FÜR EINE BIOZELLE, ELEKTRODE UND HERGESTELLTE BIOZELLE
PRODUCTION OF A BIOFILM ON AN ELECTRODE FOR A BIOCELL, ELECTRODE AND OBTAINED BIOCELL

(30) Priorité: 27.05.2008 FR 0853441
(43) Date de publication de la demande: 16.03.2011
(73) Titulaire: Centre National de la Recherche Scientifique (C.N.R.S.), 75016 Paris (FR); Université Claude Bernard Lyon I, 69100 Villeurbanne (FR); Ecole Centrale de Lyon, 69134 Ecully Cedex (FR)
(72) Inventeur: MONIER, Jean-Michel, F-69250 Neuville sur Saone (FR); HADDOUR, Naoufel, F-69100 Villeurbanne (FR); VOGEL, Timothy, F-69006 Lyon (FR)
(74) Mandataire: Lavoix
(86) Numéro de dépôt international: PCT/FR2009/050990
(87) Numéro de publication internationale: WO 2009/153499

(56) Documents cités:
- WO-A-03/097861
- WO-A-2006/072112
- WO-A-2006/099220
- WO-A-2007/131029
- DE-A1- 1 902 672
- FR-A- 2 843 490
- US-A1- 2008 090 736
- E. Baranitharan ET AL: "Effect of biofilm formation on the performance of microbial fuel cell for the treatment of palm oil mill effluent", Bioprocess and Biosystems Engineering, vol. 38, no. 1, 2 July 2014 (2014-07-02), pages 15-24, XP055216293, ISSN: 1615-7591, DOI: 10.1007/s00449-014-1239-9

## Description

La présente invention concerne un procédé de réalisation d'un biofilm à la surface d'une électrode dans un milieu contenant des bactéries, l'emploi de cette électrode dans une biopile et la production d'électricité à l'aide d'une telle biopile.

De manière très schématique, les piles mettent en oeuvre une réaction d'oxydoréduction en récupérant la part de l'énergie, autre que la chaleur, dégagée par cette réaction. On peut citer comme exemple la pile à hydrogène dans laquelle se produit une réaction d'oxydoréduction bien connue :

½ O₂ + H₂ → H₂O

Il s'agit en fait d'une combustion contrôlée de l'hydrogène et de l'oxygène. La réaction s'opère au sein d'une structure essentiellement composée de deux électrodes, l'anode et la cathode, séparées par un électrolyte. La chambre anodique est alimentée en hydrogène et la chambre cathodique en oxygène. A l'anode, l'hydrogène est oxydé en présence d'eau pour donner des protons selon la réaction :

2 H₂O + H2 → 2H₃O⁺ + 2e⁻

Les protons traversent ensuite l'électrolyte sous l'action du champ électrique et les électrons parcourent le circuit externe de la pile pour se retrouver à la cathode et réduire le dioxygène en eau selon la réaction :

½O₂ + 2H₃O⁺ + 2e⁻ → 3H₂O

Un flux d'électrons parcourt alors l'anode pour aller jusqu'à la cathode et crée ainsi un courant électrique.

Les MFC ou « Microbial fuel cells » sont des cellules électrochimiques utilisant des bactéries pour produire de l'électricité généralement à partir d'eaux usées. Plus communément appelées « biopiles » leur schéma de fonctionnement est assez similaire à celui d'une pile classique. Le métabolisme des bactéries et la dégradation de substrats se produisent également par une chaîne de réactions d'oxydoréduction. Une partie de l'énergie produite par ces réactions est récupérée dans les biopiles lesquelles produisent un courant électrique.

Traditionnellement, la biopile est composée de deux chambres contenant chacune une électrode (l'anode ou la cathode), séparées par un électrolyte, le plus souvent une membrane échangeuse d'ions. Comme dans la pile classique, la chambre cathodique est alimentée en O₂ (ou air), les protons traversent l'électrolyte et les électrons parcourent le circuit externe de la pile permettant ainsi la réduction de l'oxygène à la cathode.

Au niveau de la chambre anodique s'opère l'activité bactérienne qui permet, au final, la libération de protons et d'électrons. Cette libération est la conséquence des réactions d'oxydoréduction mises en oeuvre par les bactéries pour extraire l'énergie qui leur est nécessaire à partir des substrats à leur disposition.

L'emploi de catalyseurs onéreux à base de métaux rares (platine, or) a cependant longtemps été nécessaire pour catalyser les réactions d'oxydoréduction.

L'addition dans des piles de bactéries ou d'enzymes d'oxydoréduction particulières a été envisagée pour remplacer la catalyse par les métaux rares. Les premiers travaux ont porté sur l'emploi de bactéries capables de produire ou de régénérer le combustible, par exemple l'hydrogène, ou de régénérer la forme réduite d'un médiateur électrochimique par extraction d'électrons des substrats présents. Ces travaux n'ont cependant pas permis d'améliorer le rendement électrochimique aux électrodes et l'emploi de catalyseurs reste nécessaire.

L'article de Hasvold et collaborateurs « Sea-water battery for subsea control systems», Journal of Power Sources, 65, pages 253-261, 1997, présente une étude sur des batteries avec une anode soluble fonctionnant dans un environnement marin. Les batteries immergées dans de l'eau de mer auraient un rendement plus élevé que les batteries fonctionnant en plein air. Les auteurs ont observé la formation spontanée d'un biofilm (le terme « biofilm » signifie un film comprenant un ensemble de bactéries déposées spontanément sur une surface) à la cathode qui peut selon les cas avoir un effet négatif ou positif sur la performance de la pile. Le biofilm pourrait être à l'origine d'une amélioration de la catalyse de la réduction de l'oxygène.

WO2004/015806 propose de produire un biofilm avant de faire fonctionner la biopile. Le dispositif est un système constitué de trois électrodes, électrode de travail, électrode de référence saturée au calomel et électrode auxiliaire, et d'un potentiostat qui contrôle la différence de potentiel entre l'électrode de travail et l'électrode de référence et mesure le courant entre l'électrode de travail et l'électrode auxiliaire. Selon la différence de potentiel appliquée à l'électrode de travail, celle-ci jouera le rôle de cathode ou d'anode. Le procédé décrit est très dépendant des valeurs de potentiels régulées par le potentiostat au niveau de l'électrode du travail.

Or, lors de la polarisation et de la production d'un biofilm sur l'électrode de travail, un biofilm se forme également sur l'électrode auxiliaire et sur l'électrode de référence et ce biofilm a tendance à passiver ces électrodes. Cette passivation peut engendrer des modifications de la valeur du potentiel au niveau de l'électrode de travail et peut même court-circuiter le système.

Il a maintenant été découvert que la sélection des bactéries responsables de la catalyse anodique ou cathodique dépendait uniquement de la polarisation des électrodes et de l'intensité des champs électriques appliqués, et non pas des valeurs de potentiel au niveau des électrodes de travail.

Cette découverte permet de proposer un procédé de préparation d'un biofilm reposant sur un dispositif beaucoup plus souple et moins coûteux que celui décrit dans WO2004/015806.

Il consiste à appliquer simplement une différence de potentiel entre deux électrodes qui peuvent être de même nature. Un simple générateur électrique suffit. Ce dispositif ne nécessite l'utilisation d'aucune électrode de référence ou électrode auxiliaire (généralement du platine). L'une des deux électrodes joue le rôle d'anode et la deuxième joue le rôle de la cathode.

Ce dispositif permet ainsi de sélectionner sur l'anode un biofilm à même de catalyser l'oxydation de substrats organiques. Il permet aussi de sélectionner sur la cathode un biofilm capable de catalyser la réduction de l'oxygène. Ce dispositif est bien adapté à une application industrielle dans le sens où c'est un système simple à mettre en place et qui permet de s'affranchir de l'utilisation d'une électrode de référence et d'une électrode auxiliaire ainsi que d'un potentiostat.

La présente invention a ainsi pour objet un procédé de production d'un biofilm à la surface d'une électrode (ou d'une électrode revêtue d'un biofilm) dans un milieu liquide contenant des bactéries et un substrat permettant la croissance des bactéries, dans lequel on utilise un système d'électrodes constitué de deux électrodes, lesquelles sont reliées à une source de courant électrique continu, on place ces deux électrodes dans le milieu et l'on applique une différence de potentiel prédéterminée et constante comprise entre 100 et 700 mV entre les électrodes ce grâce à quoi des biofilms se forment à la surface des électrodes.

La présente invention a aussi pour objet un procédé de préparation d'une biopile dans lequel on prépare une électrode revêtue d'un biofilm suivant le procédé de production d'un biofilm, puis l'on emploie ladite électrode dans une biopile en tant qu'anode ou cathode.

L'invention concerne aussi un procédé de production d'électricité par biopile, dans lequel on prépare une électrode revêtue d'un biofilm suivant le procédé de production d'un biofilm, puis l'on emploie ladite électrode dans une biopile en tant qu'anode ou cathode.

Dans un mode de réalisation préféré du procédé de production d'un biofilm, le procédé vise la formation d'un biofilm à la surface de l'anode.

Le procédé de l'invention permet en effet d'obtenir de manière remarquable une anode revêtue d'un biofilm se révélant électro-attractif lorsqu'elle est transférée et/ou employée dans une biopile de type MFC.

Suivant une caractéristique de l'invention, la source de courant est un générateur de courant électrique. Ce générateur peut avoir une valeur de consigne fixe ou réglable, toutefois, la différence de potentiel ou tension appliquée est imposée, prédéterminée et elle est constante sur sa durée d'application.

Suivant une autre caractéristique de l'invention, on peut appliquer successivement des différences de potentiel déterminées différentes, par exemple croissantes ou décroissantes, chacune étant constante sur sa durée d'application.

Dans le procédé selon l'invention, le milieu contenant les bactéries est un milieu statique ou circulant. Dans un mode de réalisation préféré selon l'invention, le milieu est statique. Par statique au sens de l'invention, on entend que, pendant la préparation du biofilm, le milieu baignant l'électrode reste statique, et notamment n'est ni agité, ni soumis à une circulation.

La présente invention a plus particulièrement pour objet, un procédé dans lequel la différence de potentiel appliquée a une valeur comprise entre environ 100 mV et environ 700 mV, préférentiellement entre environ 300 mV et environ 600 mV, plus préférentiellement entre environ 450 mV et environ 550 mV. Suivant un mode de réalisation typique, la différence de potentiel est d'environ 500 mV.

Le milieu liquide employé pour la préparation du biofilm contient des bactéries et un substrat organique permettant la croissance des bactéries. Ce substrat organique peut se trouver dans le milieu liquide et/ou lui être apporté. Parmi les milieux liquides utilisables, on peut citer à titre d'exemple non limitatif les effluents industriels, les effluents domestiques, les effluents agricoles, les eaux usées, les eaux et boues de stations d'épuration, les biomasses issues de l'industrie agroalimentaire, l'eau naturelle (eau douce ou eau salée), etc.

Ce milieu peut aussi être le milieu même dans lequel la biopile sera amenée à fonctionner pour produire de l'électricité, une fois le biofilm préparé.

La durée pendant laquelle la différence de potentiel va être appliquée entre les électrodes, peut varier dans certaines proportions et selon le milieu auquel on s'adresse. Cette durée doit être suffisante pour l'obtention du biofilm. L'obtention du biofilm peut être contrôlée de la manière suivante :
- soit par la production d'électricité quand on arrête d'appliquer une tension;
- soit par l'accumulation des bactéries sur l'anode.

Dans un mode de réalisation selon l'invention, la durée durant laquelle la différence de potentiel est appliquée est comprise entre environ 3 et environ 20 jours, préférentiellement entre environ 4 et environ 7 jours.

L'anode, la cathode ou encore les deux électrodes peuvent être à base de carbone. Par matériau à base de carbone, on entend notamment le graphite et les matériaux de type fibres ou papier carbone ou carbone vitreux réticulé. D'autres matériaux peuvent être employés, par exemple acier inoxydable, aluminium, nickel ou alliages de titane. De préférence, on choisit ces matériaux sous une forme ayant une grande surface spécifique. Ainsi, lorsque le graphite est retenu, la ou les électrodes peuvent être sous forme de plaque, granule ou bâtonnet en graphite. Suivant un mode de réalisation de l'invention, les deux électrodes sont de même nature.

Le plus simple est d'effectuer la préparation du ou des biofilms dans une cuve ou analogue. Il peut néanmoins être avantageux de faire cette préparation directement dans la biopile, notamment lorsque l'on souhaite utiliser les deux électrodes ainsi préparées.

Suivant une caractéristique, le procédé de production d'un biofilm est conduit dans une biopile du type chambre double dont on a retiré ou pas encore placé la membrane de séparation. Par définition, une biopile du type chambre double comporte une chambre contenant l'anode, une chambre contenant la cathode, une membrane de séparation permettant le passage des protons mais pas celui des bactéries et du substrat organique (la membrane peut être une membrane échangeuse de cations). En l'absence de membrane, on se trouve en présence d'une chambre unique remplie du milieu liquide.

Suivant une autre caractéristique, le procédé de production d'un biofilm est conduit dans une biopile du type à chambre unique avec cathode à air. Par définition, l'anode et la cathode sont placées dans la même chambre remplie du milieu liquide, l'anode étant plongée dans la chambre sans contact avec l'air et la cathode étant placée en bordure avec une surface au contact du milieu liquide et une surface au contact de l'air extérieur. En variante, il est possible d'utiliser deux électrodes plongées dans la chambre, la cathode à air n'étant dans ce cas plus utilisée pour la préparation du biofilm.

L'efficacité d'une biopile de type MFC dépend en grande partie du transfert d'électrons depuis la bactérie adhérant à l'anode. Le problème des biopiles classiques est que la plupart des bactéries présentes dans les biofilms se trouvent être inactives du point de vue électrochimique, leurs parois cellulaires et leurs autres structures de surface n'étant pas conductrices. Justement, l'utilisation du procédé selon l'invention permet de sélectionner à l'anode un biofilm riche en bactéries électro-attractives à fort pouvoir redox. La valeur du potentiel redox du biofilm ainsi obtenu, lorsqu'il est placé dans les conditions de fonctionnement de la biopile, est supérieur au potentiel d'un biofilm normal sans application du procédé de l'invention (c'est-à-dire si l'on installe simplement la biopile de type MFC au contact du même milieu, et l'on mesure le pouvoir redox une fois que la biopile a atteint son équilibre de fonctionnement). La valeur du potentiel redox peut être mesurée par voltammétrie (en anglais « voltammetry ») cyclique (par exemple avec une électrode de référence au chlorure d'argent). Il s'ensuit que les biopiles employant une anode selon l'invention développent une puissance électrique supérieure par rapport à une biopile dont l'anode n'aurait pas été enrichie conformément à l'invention.

La présente invention a également pour objet une telle anode revêtue d'un biofilm comportant ou constitué de bactéries à potentiel redox élevé obtenu par la mise en oeuvre du procédé se!on l'invention. Ce biofilm est à même de catalyser l'oxydation de substrats organiques.

La présente invention a également pour objet une cathode revêtue d'un biofilm constitué de bactéries et capable de catalyser la réduction de l'oxygène.

La présente invention a également pour objet une biopile comprenant une électrode revêtue d'un biofilm et obtenue par la mise en oeuvre du procédé de préparation de biofilm selon l'invention.

Suivant un premier mode de réalisation, la biopile contient une anode selon l'invention revêtue de son biofilm conforme à l'invention.

Suivant un deuxième mode de réalisation, la biopile contient une cathode selon l'invention revêtue de son biofilm conforme à l'invention.

Suivant un troisième mode de réalisation, la biopile contient une anode et une cathode selon l'invention, revêtues de leur biofilm conforme à l'invention.

Suivant une modalité, la biopile est du type à chambre double avec séparation par membrane. Dans un mode de réalisation avantageux, l'anode, la cathode ou les deux électrodes ont été préparées dans la biopile en l'absence de membrane de séparation, et cette dernière est ajoutée pour former la biopile complète.

Suivant une autre modalité, la biopile est du type à chambre unique avec cathode à air. Dans un mode de réalisation avantageux, l'anode, la cathode ou les deux électrodes ont été préparées dans la biopile elle-même.

Les biopiles ainsi obtenues par la mise en oeuvre du procédé de l'invention, trouvent leur application dans les installations de production d'électricité à partir de milieux d'origines divers contenant des substrats métabolisables par les bactéries présentes à la surface de la ou des électrodes. Ces milieux peuvent être liquides, par exemple les effluents industriels, les effluents domestiques, les effluents agricoles, les eaux usées, les eaux et boues de stations d'épuration, les biomasses issues de l'industrie agroalimentaire, l'eau naturelle (eau douce ou eau salée), ou solide, par exemple sols, sédiments marins ou fluviaux, etc.

Suivant une modalité particulière, le dispositif qui a servi à la préparation de biofilm est constitué de la biopile elle-même. Dans ce cas, après ouverture ou déconnexion de la source de courant, éventuellement ajout de la membrane de séparation s'il s'agit d'une biopile à chambre double, la biopile est mise à fonctionner en générateur de courant.

La configuration des biopiles est par ailleurs connue de l'homme du métier. Ces biopiles comportent des ouvertures pour que le milieu apporteur de substrat organique puisse entrer en contact avec l'anode. Une circulation peut être prévue pour assurer cette mise en contact.

Un autre objet de l'invention est une installation de production d'électricité comprenant une ou plusieurs biopiles telles que décrites ci-dessus. Lorsque plusieurs biopiles sont présentes, elles peuvent être montées en série ou en parallèle.

La présente invention va être illustrée à travers les exemples suivants sans toutefois en limiter la portée, et en se référant aux dessins annexés.
La figure 1 présente schématiquement un dispositif électrochimique permettant de produire des biofilms anodique et cathodique, la partie gauche de la figure présentant le dispositif à sa mise en route et la partie droite présentant le dispositif après formation d'un biofilm à la surface des électrodes.
La figure 2 est un voltammogramme cyclique en tampon phosphate (0,1 M, pH=7) d'un biofilm formé sur l'anode.
La figure 3 est un voltammogramme cyclique en tampon phosphate (0,1 M, pH=7) d'un biofilm formé sur la cathode.
La figure 4 est un voltammogramme cyclique en tampon phosphate (0,1 M, pH=7) d'un biofilm formé sur une électrode témoin non polarisée.
La figure 5 présente schématiquement une biopile.
La figure 6 est un graphe montrant la tension d'une biopile selon l'invention versus une biopile sans prétraitement anodique, en fonction du temps.

### Exemple 1 : Préparation d'un biofilm à l'anode et à la cathode

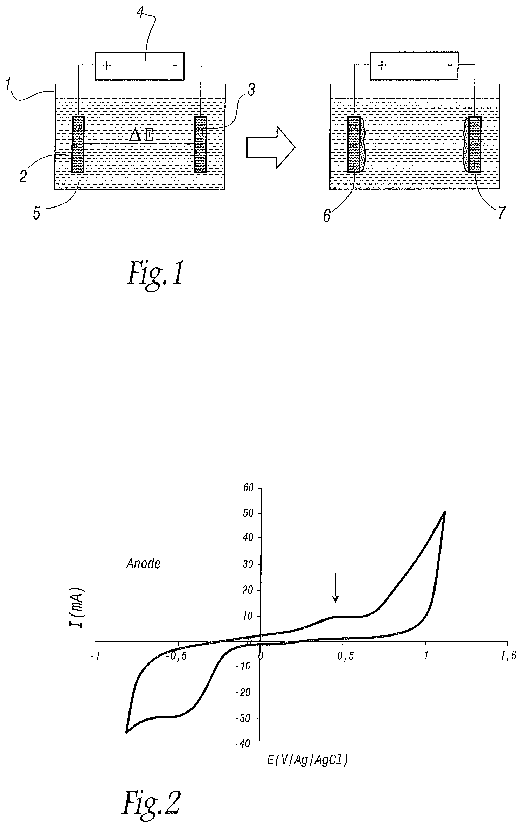

Le dispositif électrochimique représenté à la figure 1 comprend une cuve 1, deux électrodes similaires en graphite (anode 2 et cathode 3) et un générateur de courant électrique 4. La cuve est remplie d'eaux usées 5 provenant d'une installation de retraitement des eaux usées domestiques.

A l'aide du générateur 4, on a appliqué une tension de 0,5 V pendant 4 jours. La partie gauche de la figure 1 présente le dispositif à sa mise en route et la partie droite présente le dispositif après ces 4 jours, à l'issue desquels un biofilm 6 s'est formé à l'anode et un biofilm 7 s'est formé à la cathode.

Par comparaison, on a laissé un dispositif similaire 4 jours sans appliquer de tension (électrodes non polarisées).

On a ensuite déterminé les voltammogrammes cycliques en tampon phosphate (0,1 M, pH=7) des biofilms qui se sont formés à l'anode, à la cathode et sur une électrode non polarisée. Pour ce faire on réalise une voltammétrie à l'aide d'un dispositif classique à trois électrodes, dont une électrode de référence au chlorure d'argent.

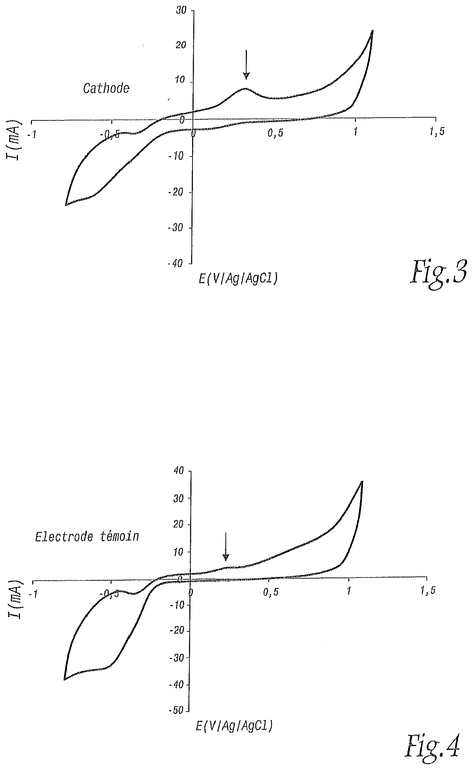

La comparaison des voltammogrammes démontre clairement l'obtention de biofilms ayant des activités électrochimiques différentes (figures 2, 3 et 4). Le pic de potentiel positif du biofilm anodique se situe aux environs de 0,46 V/Ag/AgCl et est supérieur à ceux observés pour la cathode (0,31 V/Ag/AgCl) et pour l'électrode non polarisée (0,25 V/Ag/AgCl). La variance de la position des pics anodiques reflète la différence en énergie potentielle entre des donneurs d'électron microbiens, tels que des protéines redox, pour chaque biofilm et démontre qu'une polarisation positive selon l'invention d'une électrode lors de la formation d'un biofilm permet d'enrichir celui-ci en bactéries ayant un fort potentiel redox.

### Exemple 2 : Biopile

On a utilisé une biopile à cathode à air. Elle comprend une enceinte 8 renfermant une anode 9 et une cathode 10 placée au niveau d'une paroi de l'enceinte de manière à présenter une surface tournée vers l'intérieur de l'enceinte et une surface tournée vers l'extérieur et en contact avec l'air. On a figuré un circuit électrique réunissant les électrodes et comprenant une résistance 11, aux bornes de laquelle on mesure la tension de la biopile à l'aide d'un dispositif de mesure non représenté.

L'enceinte 8 et la cathode 10 délimitent une chambre, laquelle est remplie avec une solution nutritive minimum : tampon phosphate (PBS) : Na₂HPO₄ (4,1 g/l) et NaH₂PO₄, H₂0 (2,9 g/l), NH₄Cl (0,3 g/l), KCL (0,1 g/l) ; et substrat : acétate (>1,0 g/l).

Deux biopiles ont ainsi été préparées, l'une dans laquelle l'anode est une anode préparée selon l'exemple 1 et recouverte d'un biofilm 13, l'autre dans laquelle l'anode n'a subi aucun prétraitement.

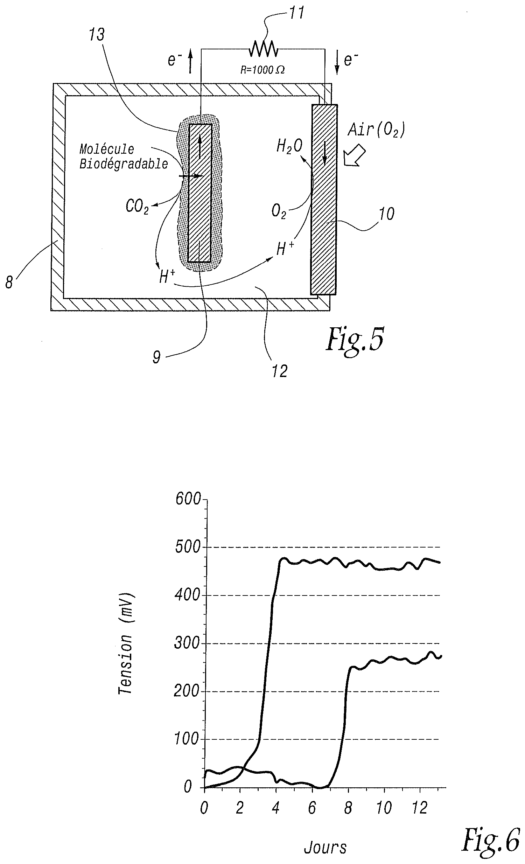

La figure 6 est un graphe montrant la tension de ces biopiles, en fonction du temps. La courbe correspondant à l'apparition la plus précoce d'une tension aux bornes et du plateau le plus élevé correspond à la biopile équipée d'une anode à biofilm, obtenue selon l'exemple 1. Une tension est observée très rapidement avec la biopile selon l'invention. De même, la tension maximale de 470 mV environ est très supérieure aux 270 mV de la biopile sans prétraitement à l'anode. Ces résultats démontrent que la formation d'un biofilm à l'anode selon l'invention améliore nettement les performances d'une biopile grâce à l'enrichissement électrochimique du biofilm en bactéries efficaces par polarisation positive à l'anode.

## Revendications

1. Procédé de production d'une électrode de biopile, recouverte à sa surface d'un biofilm, dans un milieu liquide contenant des bactéries et un substrat permettant la croissance des bactéries, dans lequel on utilise un système d'électrodes constitué de deux électrodes, lesquelles sont reliées à une source de courant électrique continu, on place ces deux électrodes dans le milieu et l'on applique une différence de potentiel prédéterminée et constante comprise entre 100 et 700 mV entre les électrodes ce grâce à quoi des biofilms se forment à la surface des électrodes.

2. Procédé selon la revendication 1, dans lequel la différence de potentiel appliquée a une valeur comprise entre 300 mV et 600 mV.

3. Procédé selon la revendication 1, dans lequel la différence de potentiel appliquée a une valeur comprise entre 450 mV et 550 mV.

4. Procédé selon l'une quelconque des revendications 1 à 3, dans lequel la différence de potentiel est appliquée sur une durée comprise entre 3 et 20 jours.

5. Procédé selon la revendication 4, dans lequel cette durée est comprise entre 4 et 7 jours.

6. Procédé selon l'une quelconque des revendications 1 à 5, dans lequel les électrodes sont de même nature.

7. Procédé selon la revendication 6, dans lequel l'anode et/ou la cathode est/sont à base de carbone et/ou d'acier inoxydable, d'aluminium, de nickel ou d'alliages de titane.

8. Procédé de préparation d'une biopile, comprenant la préparation d'une électrode suivant le procédé selon l'une quelconque des revendications 1 à 7, puis l'emploi de cette électrode dans un biopile en tant qu'anode ou cathode.

9. Procédé de production d'électricité dans lequel on fait fonctionner une biopile telle qu'obtenue à la revendication 8.

10. Installation de production d'électricité, comprenant une ou plusieurs biopiles préparées selon la revendication 8.

11. Electrode revêtue d'un biofilm, obtenue par la mise en oeuvre du procédé selon l'une quelconque des revendications précédentes 1 à 7.

12. Anode revêtue d'un biofilm contenant des bactéries à fort pouvoir redox, obtenue selon l'une quelconque des revendications précédentes 1 à 7.

13. Biopile comprenant une électrode obtenue par la mise en oeuvre du procédé selon l'une quelconque des revendications précédentes.

14. Biopile comprenant une anode selon la revendication 12.

## Patentansprüche

1. Verfahren zur Herstellung einer Bio-Brennstoffzelle-Elektrode, welche auf ihrer Oberfläche mit einem Biofilm bedeckt ist, in einem flüssigen Milieu, das Bakterien und ein Substrat, dass das Wachstum der Bakterien erlaubt, aufweist, wobei ein Elektrodensystem, das aus zwei Elektroden, welche mit einer elektrischen Gleichstromquelle verbunden sind, gebildet ist, verwendet wird, diese zwei Elektroden in dem Milieu platziert werden und eine vorbestimmte und konstante Potentialdifferenz, die zwischen 100 und 700 mV liegt, zwischen den Elektroden angelegt wird, wodurch sich Biofilme auf der Oberfläche der Elektroden bilden.

2. Verfahren gemäß Anspruch 1, wobei die angelegte Potentialdifferenz einen Wert zwischen 300 mV und 600 mV hat.

3. Verfahren gemäß Anspruch 1, wobei die angelegte Potentialdifferenz einen Wert zwischen 450 mV und 550 mV hat.

4. Verfahren gemäß irgendeinem der Ansprüche 1 bis 3, wobei die Potentialdifferenz über einen Zeitraum, der zwischen 3 und 20 Stunden liegt, angelegt wird.

5. Verfahren gemäß Anspruch 4, wobei der Zeitraum zwischen 4 und 7 Stunden liegt.

6. Verfahren gemäß irgendeinem der Ansprüche 1 bis 5, wobei die Elektroden von derselben Art sind.

7. Verfahren gemäß Anspruch 6, wobei die Anode und/oder die Kathode auf Basis von Kohlenstoff und/oder rostfreiem Stahl, Aluminium, Nickel oder Titanlegierungen ist/sind.

8. Verfahren zur Vorbereitung einer Bio-Brennstoffzelle, aufweisend das Vorbereiten einer Elektrode nach dem Verfahren gemäß irgendeinem der Ansprüche 1 bis 7, danach das Verwenden dieser Elektrode in einer Bio-Brennstoffzelle als Anode oder Kathode.

9. Verfahren zum Erzeugen von Elektrizität, wobei eine in Anspruch 8 erhaltene Bio-Brennstoffzelle betrieben wird.

10. Einrichtung zur Erzeugung von Elektrizität, welche eine oder mehrere gemäß Anspruch 8 vorbereitete Bio-Brennstoffzellen aufweist.

11. Mit einem Biofilm beschichtete Elektrode, welche ferner durch Umsetzen des Verfahrens gemäß irgendeinem der vorhergehenden Ansprüche 1 bis 7 erhalten ist.

12. Mit einem Biofilm, der Bakterien mit starker Redoxfähigkeit aufweist, beschichtete Anode, die gemäß irgendeinem der vorhergehenden Ansprüche 1 bis 7 erhalten ist.

13. Bio-Brennstoffzelle, welche eine durch Umsetzen des Verfahrens gemäß irgendeinem der vorhergehenden Ansprüche erhaltene Elektrode aufweist.

14. Bio-Brennstoffzelle, welche eine Anode gemäß Anspruch 12 aufweist.

## Claims

1. A process for producing an electrode of a biofuel cell recovered on its surface by a biofilm, in a liquid medium containing bacteria and a substrate allowing the bacteria to grow, in which a system of electrodes is used that consists of two electrodes which are connected to a DC current source, these two electrodes are placed in the medium and a predetermined constant potential difference comprised between 100 and 700 mV is applied between the electrodes, thereby forming biofilms on the surface of the electrodes.

2. The process as claimed in claim 1, in which the potential difference applied is between 300 mV and 600 mV.

3. The process as claimed in claim 1, in which the potential difference applied is between 450 mV and 550 mV.

4. The process as claimed in any one of claims 1 to 3, in which the potential difference is applied for a length of time of between 3 and 20 days.

5. The process as claimed in claim 4, in which this length of time is between 4 and 7 days.

6. The process as claimed in any one of claims 1 to 5, in which the electrodes have the same nature.

7. The process as claimed in claim 6, in which the anode and/or the cathode are/is based on carbon and/or stainless steel, aluminum, nickel or on titanium alloys.

8. A process for preparing a biofuel cell comprising the preparation of an electrode according to the process as claimed in any one of claims 1 to 7 and then the use of this electrode in a biofuel cell as either anode or cathode.

9. A process for producing electricity, in which a biofuel cell such as that obtained in claim 8 is used.

10. An electricity production facility comprising one or more biofuel cell(s) prepared according to claim 8.

11. An electrode coated with a biofilm that is obtained by implementing the process as claimed in any one of claims 1 to 7.

12. An anode coated with a biofilm, containing highly oxidizing/reducing bacteria, that is obtained according to any one of claims 1 to 7.

13. A biofuel cell comprising an electrode obtained by implementing the process as claimed in any one of the preceding claims.

14. A biofuel cell comprising an anode as claimed in claim 12.
